# EUROPEAN PATENT APPLICATION

(11) **EP 4 837 626 A1**
(43) Date of publication of application: **11.11.2026**
(21) Application number: 24915476.6
(22) Date of filing: 14.11.2024
(51) Int. Cl.: A61N 2/02, A61N 2/00, H01F 27/28

(54) **PAD-TYPE MAGNETIC STIMULATION DEVICE**

(30) Priority: 05.01.2024 KR 20240002256
(71) Applicant: Remed Brainstim Co., Ltd., Seongnam-si Gyeonggi-do 13647 (KR)
(72) Inventor: YOON, Se Jin, Anyang-si, Gyeonggi-do 14055 (KR); SHIN, Hee Joon, Seoul 07210 (KR); KIM, Il Kyu, Suwon-si, Gyeonggi-do 16354 (KR); LEE, Byung Ju, Seoul 08651 (KR)
(74) Representative: ABG Intellectual Property Law, S.L.
(86) International application number: PCT/KR2024/096566
(87) International publication number: WO 2025/147066

(57) **Abstract**

The present invention relates to a pad-type magnetic stimulation device for providing non-invasive treatment by applying a magnetic field to nerve cells or muscle cells of the body. The pad-type magnetic stimulation device of the present invention comprises: a pad-type case unit which includes an upper case unit including a body contact surface and a lower case unit coupled to the lower portion of the upper case unit to form an inner space; a magnetic field generating coil included in the inner space to generate a magnetic field; a cooling unit which cools the magnetic field generating coil by generating an air flow in the inner space; and a guide unit which provides a space in which the magnetic field generating coil is seated, and guides the air flow in the vicinity of the magnetic field generating coil.

## Description

### TECHNICAL FIELD

The present invention relates to a pad-type magnetic stimulation device, and more particularly, to a pad-type magnetic stimulation device that provides non-invasive treatment by applying a magnetic field to nerve cells or muscle cells of the body.

### BACKGROUND ART

Neuromagnetic stimulation (NMS) or pulsed electromagnetic field (PEMF) treatment is a method used to treat inflammation, swelling, and pain caused by damage of soft tissues such as skin, muscles, tendons, and ligaments due to musculoskeletal disorders. An electromagnetic field is directly transmitted to the damaged tissue to induce microcurrents, thereby promoting recovery of the damaged cells. In addition, core-muscle strength magnetic stimulation (CSMS) is a method for strengthening core muscles by applying high-frequency magnetic stimulation to repeat muscle contraction and relaxation more than 40 times per second.

To perform the various types of magnetic stimulation treatments, a position of a stimulator including a coil having a certain diameter gradually moves to find a point at which the greatest stimulation occurs. Thereafter, a stimulation intensity is determined based on a threshold so that a corresponding portion is repeatedly stimulated.

Conventionally, development of a structure of increasing in strength of a magnetic field and focusing the magnetic field on a desired target area has continued through structural designs of various magnetic field generating coils. Since the electrical activity induced from the coil is highly focused and attenuated very quickly away from a center of the coil, coil design technologies and the development of coil technology with a structure for proper contact with the target area are important for optimal stimulation.

In addition, the recommended treatment time for pulsed electromagnetic field (PEMF) is 5 days per week, once a day, and about 20 minutes, and thus, frequent treatments are required. For example, it has been reported that nerve regeneration and functional pain relief effects are observed when the magnetic stimulation treatment, which is performed for about 20 minutes to about 50 minutes per day, is repeatedly applied to patients with chronic pain over several days. In various experimental studies, it has been demonstrated that PEMF exerts a significant impact on physiological processes. Particularly, it is known to enhance metabolism of fibroblasts, chondrocytes, and osteocytes, as well as to modulate effects of hormones and neurotransmitters on receptors of various cells.

However, since existing PEMF magnetic stimulation devices are non-movable, it is difficult to match this treatment cycle to the domestic medical market situation. Thus, there is a need to introduce a miniaturized PEMF treatment device that is capable of being easily used at home.

### DISCLOSURE OF THE INVENTION

### TECHNICAL PROBLEM

The present invention is invented to overcome the above-described problems, and discloses a magnetic stimulation device provided with an applicator in the form of a pad that is movable.

In addition, the present invention discloses a pad-type magnetic stimulation device provided with a magnetic field generating unit that generates a magnetic field to apply the magnetic field to a body portion, and a cooling unit that cools heat generated from the magnetic field generating unit.

In addition, the present invention discloses a pad-type magnetic stimulation device that facilitates air circulation by providing a flow path as a cooling unit and efficiently controls heat generation of a magnetic field generating unit to optimize cooling performance.

### TECHNICAL SOLUTION

A pad-type magnetic stimulation device of the present invention for achieving the above objects is provided as a pad-type magnetic stimulation device, which applies a magnetic field to nerve cells or muscle cells of a body to provide non-invasive stimulation, including: a pad-type case unit including an upper case unit having a body contact surface and a lower case unit coupled to a lower portion of the upper case unit to form an inner space; a magnetic field generating coil provided in the inner space to generate a magnetic field; a cooling unit configured to cool the magnetic field generating coil by generating an airflow in the inner space; and a guide unit configured to provide a space, in which the magnetic field generating coil is seated, and guide the airflow in the vicinity of the magnetic field generating coil.

In addition, in the pad-type magnetic stimulation device, the guide unit may be configured to guide air introduced from a first side surface of the lower case unit so that the air sequentially flows over an upper surface of the magnetic field generating coil and a lower surface of the magnetic field generating coil and then is discharged to a second side surface of the lower case unit.

In addition, in the pad-type magnetic stimulation device, the guide unit may include: a bottom surface on which the magnetic field generating coil is seated; a first vertical part which is formed to have a predetermined height along an outer circumference of the bottom surface and surrounds an outer circumference of the magnetic field generating coil; and a second vertical part which is formed to have a predetermined height at a central area of the bottom surface and is configured to support an inner circumference of the magnetic field generating coil.

In addition, in the pad-type magnetic stimulation device, the guide unit may include a protruding rib protruding from the bottom surface to support the lower surface of the magnetic field generating coil so that the magnetic field generating coil is spaced apart from the bottom surface.

In addition, in the pad-type magnetic stimulation device, the guide unit may include a through-hole formed to pass through the bottom surface in at least partial area of the bottom surface.

In addition, in the pad-type magnetic stimulation device, the cooling unit may include: a first vent formed at one side of the lower case unit to introduce external air into the inner space; a second vent formed at the other side of the lower case unit to discharge the air in the inner space to the outside; and an air blowing device disposed at a side of the second vent to discharge the air in the inner space, wherein the air blowing device may be disposed below the through-hole.

In addition, in the pad-type magnetic stimulation device, when the upper case unit and the lower case unit are coupled to each other, the second vertical part may be in contact with a rear surface of the upper case unit.

### ADVANTAGEOUS EFFECTS

As described above, the pad-type magnetic stimulation device of the present invention may be provided with the magnetic field generating unit that generates the magnetic field and the cooling unit that cools the heat generated from the magnetic field generating unit to allow the user to perform the PEMF stimulation for the pain relief or muscle strength enhancement without being restricted by the time or space.

In addition, according to the pad-type magnetic stimulation device of the present invention, the flow path may be provided as the cooling unit to facilitate the circulation of the external air and efficiently control the heat generation of the magnetic field generating unit, thereby optimizing the cooling performance for cooling the heat of the magnetic field.

In addition, the present invention may significantly improve the cooling efficiency on the area between the magnetic field generating unit and the body portion of the user to significantly improve the heat generation control performance of the magnetic stimulation device at the portion adjacent to the human body.

In addition, the magnetic field generating coil included in the pad-type magnetic stimulation device of the present invention may use the oxygen free copper (OFC) coil, which minimizes the elements that interrupt the flow of the current, to reduce the impedance of the coil, thereby reducing the heat generated from the coil and maximizing the magnetic field generation efficiency.

### BRIEF DESCRIPTION OF THE DRAWINGS

FIG. 1 is a perspective view illustrating a state when a pad-type magnetic stimulation device is viewed from above according to an embodiment of the present invention.
FIG. 2 is a perspective view illustrating a state when the pad-type magnetic stimulation device is viewed from below according to an embodiment of the present invention.
FIG. 3 is an exploded perspective view of the pad-type magnetic stimulation device according to an embodiment of the present invention.
FIG. 4 is a perspective view of a guide unit included in the pad-type magnetic stimulation device of the present invention.
FIG. 5 is a schematic cross-sectional view for explaining a flow of air that is introduced into and discharged from the pad-type magnetic stimulation device of the present invention.
FIG. 6 is a schematic cross-sectional view for explaining a flow of air that is introduced into and discharged from the pad-type magnetic stimulation device of the present invention.
FIG. 7 is a perspective view of a magnetic field generating coil included in the pad-type magnetic stimulation device of the present invention.
FIG. 8 is a cross-sectional view taken along line A-A' of the magnetic field generating coil of FIG. 7.
FIG. 9 is a cross-sectional view taken along line B-B' of the magnetic field generating coil of FIG. 7.
FIG. 10 is a cross-sectional view taken along line C-C' of the magnetic field generating coil of FIG. 7.
FIG. 11 is a schematic view for explaining a structure of the magnetic field generating coil.
FIG. 12 is a graph obtained by comparing results of experiments on heat temperatures of a magnetic field generating coil included in the present invention with those of different type of magnetic field generating coil.

### MODE FOR CARRYING OUT THE INVENTION

Advantages and features of the present disclosure, and implementation methods thereof will be clarified through following examples described with reference to the accompanying drawings. The present invention may, however, be embodied in different forms and should not be construed as limited to the embodiments set forth herein. Rather, these embodiments are provided so that this disclosure will be thorough and complete, and will fully convey the scope of the present invention to those skilled in the art. Further, the present invention is only defined by scopes of claims.

Although the terms first, second, etc. are used to describe various components, these components are not limited by these terms. These terms are only used to distinguish one component from another component. Accordingly, a first component that will be described below may be a second component within the technical idea of the present disclosure.

In the embodiments below, the terms such as "include" or "have" mean that a feature or component described in the specification is present, and do not exclude in advance the possibility that one or more other features or components may be added.

In the drawings, for convenience of description, the dimensions of elements are exaggerated or downscaled. For example, since the size and shape of each of the components illustrated in the drawings are arbitrarily shown for convenience of description, the present invention is not necessarily limited to the illustrated.

Like reference numerals refer to like elements throughout.

Each feature of the various embodiments of the present invention can be partially or fully coupled or combined with each other, and as can be fully understood by those skilled in the art, various technical interconnections and operations are possible. Also, the embodiments may be independently performed with respect to each other or performed in combination of each other.

Hereinafter, a pad-type magnetic stimulation device 1000 of the present invention will be described in detail with reference to the attached drawings.

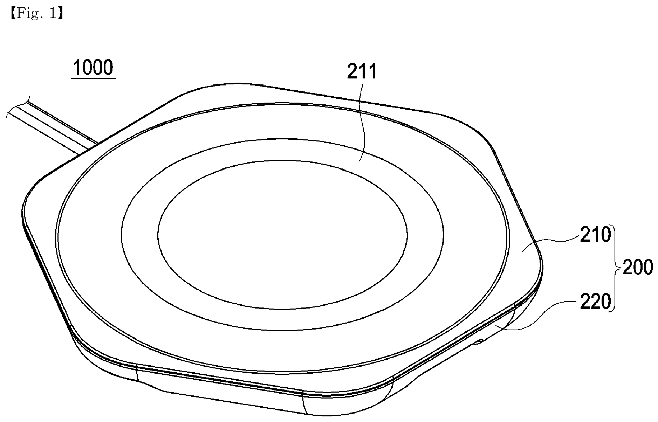

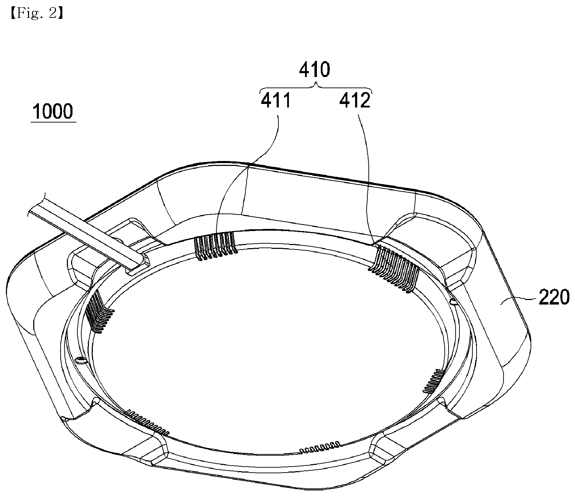

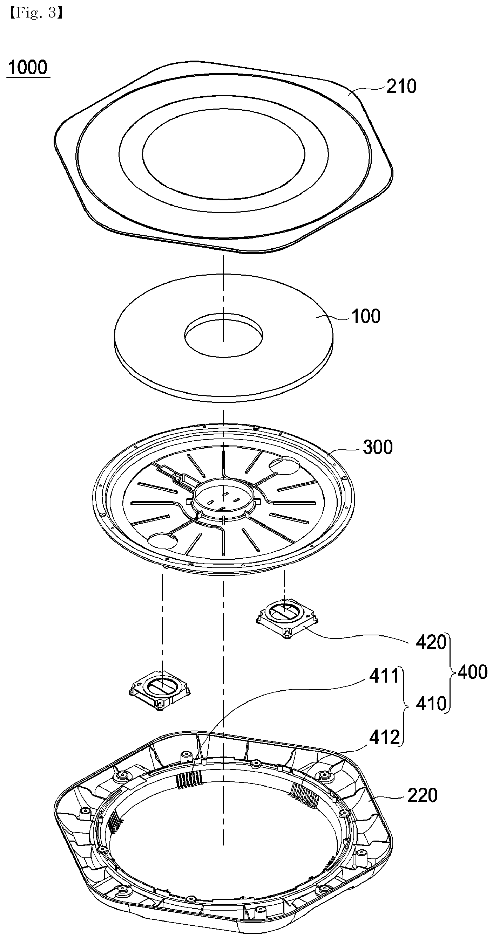

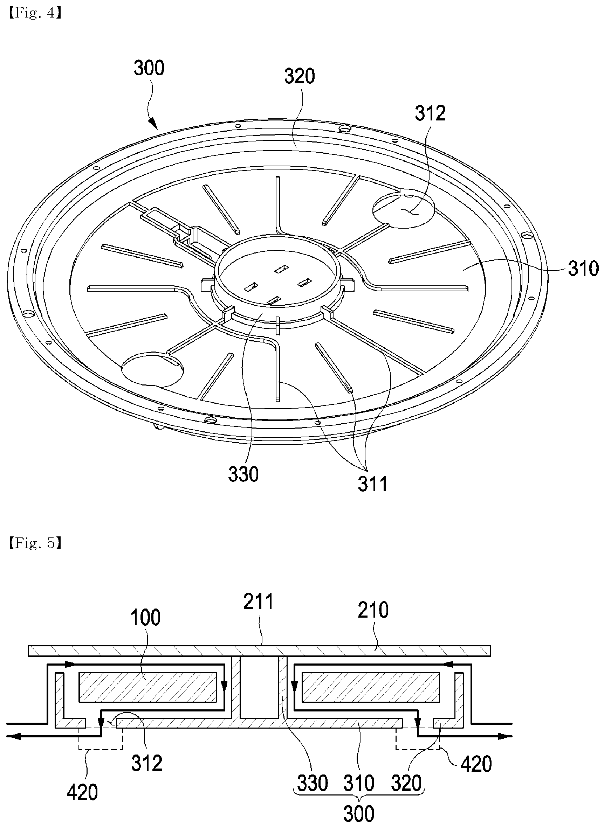

FIG. 1 is a perspective view illustrating a state when a pad-type magnetic stimulation device 1000 is viewed from above according to an embodiment of the present invention, FIG. 2 is a perspective view illustrating a state when the pad-type magnetic stimulation device 1000 is viewed from below according to an embodiment of the present invention, FIG. 3 is an exploded perspective view of the pad-type magnetic stimulation device 1000 according to an embodiment of the present invention, and FIG. 4 is a perspective view of a guide unit 300 included in the pad-type magnetic stimulation device 1000 of the present invention.

The pad-type magnetic stimulation device 1000 of the present invention is a device that provides non-invasive stimulation by applying a magnetic field to nerve cells or muscle cells of the body. The pad-type magnetic stimulation device 1000 of the present invention may be a pulsed electromagnetic field (PEMF) device, a neuro magnetic stimulation (NMS) device, or a core-muscle strength magnetic stimulation (CSMS) device.

The pad-type magnetic stimulation device 1000 of the present invention includes a magnetic field generating coil 100, a case unit 200, a guide unit 300, and a cooling unit 400.

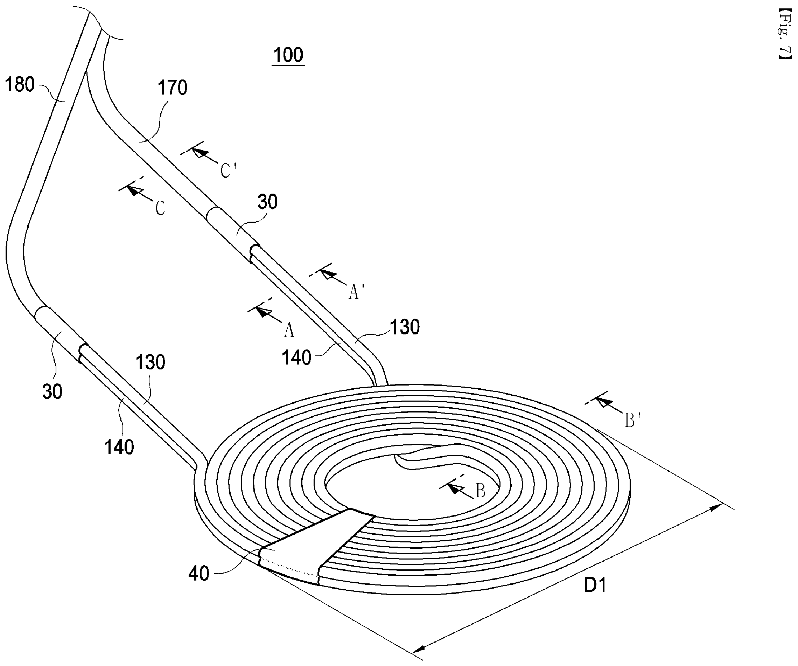

The magnetic field generating coil 100 is configured to generate a magnetic field and is a coil wound to have a predetermined diameter. The magnetic field generating coil 100 generates the magnetic field to apply the magnetic field to nerve or muscle cells of the body. A specific structure and material of the magnetic field generating coil 100 will be described below with reference to FIGS. 7 to 12.

The case unit 200 is configured to form an outer appearance of an applicator of the magnetic stimulation device 1000 according to the present invention and has a pad-like shape as illustrated in FIGS. 1 and 2. The case unit 200 includes an upper case unit 210 and a lower case unit 220. The case unit 200 includes an upper case unit 210 including a body contact surface 211, and a lower case unit 220 coupled to a lower portion of the upper case unit 210 to form an inner space. The above-described magnetic field generating coil 100 is included in the inner space formed by the case unit 200.

Although the case unit 200 is exemplarily illustrated as having an overall hexagonal shape, the scope of the present invention is not limited to the shape, and various pad shapes such as circular or polygonal shapes may be applied. The case unit 200 has an overall flat and wide shape, and the upper case unit 210 is provided flatly to provide a space in which a portion of a user's body is seated.

The body contact surface 211 included in the upper case unit 210 is a surface for seating the body portion of the user for which pain treatment or the like is to be performed. The body contact surface 211 may correspond to an area on which the magnetic field generated from the generating coil 100 is focused.

In the magnetic stimulation device 1000, it is important to cool an area between the magnetic field generating coil 100 that generates the magnetic field and the contact portion with the user's body. In other words, it is important to develop a technology for effectively cooling the area between the magnetic field generating coil 100 and the body contact surface 211. The body contact surface 211 should be preferably controlled at a temperature of below 40 degrees Celsius, even if the temperature of the magnetic field generating coil 100 rises. The present invention enables the efficient cooling at a side of the body contact surface 211 through the structure and arrangement of the guide unit 300 and the cooling unit 400.

The guide unit 300 provides the space in which the magnetic field generating coil 100 is seated and guides a flow of air around the magnetic field generating coil 100.

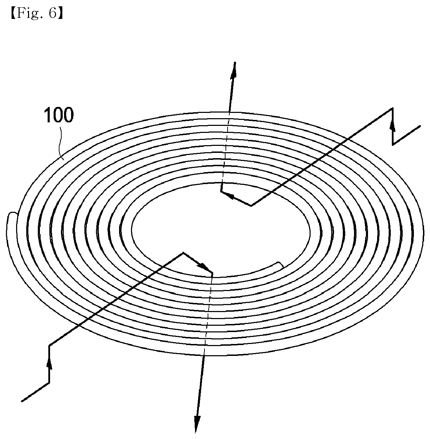

The guide unit 300 guides the air that is introduced from a first side surface of the lower case unit 220 so that the air sequentially flows over an upper surface of the magnetic field generating coil 100 and a lower surface of the magnetic field generating coil 100 and then is discharged to a second side surface of the lower case unit 220. A specific circulation path of the air will be described below with reference to FIGS. 5 and 6.

The guide unit 300 includes a bottom surface 310, a first vertical part 320, and a second vertical part 330. As illustrated in FIG. 4, the guide unit 300 may have a shape such as an approximately plate shape.

The bottom surface 310 of the guide unit 300 is a surface on which the magnetic field generating coil 100 is seated, and it is preferable that the bottom surface 310 is formed to correspond to a shape and size of the magnetic field generating coil 100. For example, the bottom surface 310 may be formed in a circular shape.

The first vertical part 320 of the guide unit 300 is formed to have a predetermined height along an outer circumference of the bottom surface 310. The first vertical part 320 is formed to surround the outer circumference of the magnetic field generating coil 100 when the magnetic field generating coil 100 is seated on the bottom surface 310.

The first vertical part 320 is a portion that guides the air introduced from the lower case unit 220 so that the air flows toward the upper surface of the magnetic field generating coil 100. The first vertical part 320 is formed at a height higher than that of the bottom surface 310, and it is preferably that the first vertical part 320 is formed to have a height corresponding to the upper surface of the magnetic field generating coil 100 when the magnetic field generating coil 100 is seated thereon.

The second vertical part 330 of the guide unit 300 is formed to have a predetermined height at a central region of the bottom surface 310. The second vertical part 330 is formed to support an inner circumference of the magnetic field generating coil 100 when the magnetic field generating coil 100 is seated on the bottom surface 310.

The second vertical part 330 is a portion that guides the air that is introduced toward the upper surface of the magnetic field generating coil 100 so that the air moves from the central area of the magnetic field generating coil 100 toward the lower surface of the magnetic field generating coil 100. The second vertical part 330 is formed at a height higher than that of the bottom surface 310, and it is preferable that the second vertical part 330 is formed to be higher level than the upper surface of the magnetic field generating coil 100 when the magnetic field generating coil 100 is seated thereon. When the upper case unit 210 and the lower case unit 220 are coupled to each other, the second vertical part 330 may be in contact with a rear surface of the upper case unit 210.

The guide unit 300 includes a plurality of protruding ribs 311 formed on the bottom surface 310. Each of the protruding ribs 311 protrudes from the bottom surface 310 to support the lower surface of the magnetic field generating coil 100 so that the magnetic field generating coil 100 is spaced apart from the bottom surface 310.

Furthermore, the protruding rib 311 forms a passage for the air that is introduced toward the lower surface of the magnetic field generating coil 100. The air that is introduced toward the bottom surface of the magnetic field generating coil 100 may sweep across the lower surface through the plurality of protruding ribs 311. The plurality of protruding ribs 311 may be provided to increase in time during which the air for cooling remains near the magnetic field generating coil 100, thereby extending a heat exchange time for the cooling.

In addition, the guide unit 300 includes a through-hole 312 formed to pass through the bottom surface 310 in at least partial area of the bottom surface 310.

The through-hole 312 is configured to discharge heated air that has completed heat exchange at the bottom surface of the magnetic field generating coil 100 to the outside. Referring to FIG. 3, an air blowing device 420 such as a fan is disposed below the through-hole 312.

The cooling unit 400 generates an airflow within the inner space formed by the case unit 200 to cool the heat generated from the magnetic field generating coil 100. The cooling unit 400 includes a vent 410 through which the air is introduced and discharged between external and inner spaces of the device, and the air blowing device 420 that generates the airflow.

Particularly, the vent 410 may include a first vent 411 through which external air is introduced into the inner space, and a second vent 412 through which the air in the inner space is discharged to the outside. The first vent 411 and the second vent 412 are distinguished by its function, but may be identical in form. In addition, positions of the first vent 411 and the second vent 412 may vary depending on the arrangement of the air blowing device 420 and are not limited to the positions illustrated in FIGS. 2 and 3.

The first vent 411 is formed at one side of the lower case unit 220 and allows the external air to be introduced into the inner space of the case unit 200. The first vent 411 includes a plurality of holes that pass through the lower case unit 220. The second vent 412 is formed at the other side of the lower case unit 220 and allows the air in the inner space to be discharged to the outside of the device. The second vent 412 includes a plurality of holes that pass through the lower case unit 220.

Here, one side and the other side of the lower case unit 220 refer to positions different from each other and are not limited to positions that are opposite to or face each other. Although the first vent 411 and the second vent 412 are illustrated as forming an angle of approximately 45 degrees therebetween, this is merely exemplary positions. It means that the first vent 411 and the second vent 412 are formed at positions spaced apart from each other.

The air blowing device 420 is disposed at a side of the second vent 412. The air blowing device 420 may be a fan that induces air circulation, and other configurations such as a blower may also be applied. The air blowing device 420 may be disposed at a side of the second vent 412 to discharge the heated air from the inner space to the outside.

Hereinafter, the circulation path for the cooling air through the guide unit 300 and the cooling unit 400 will be described in more detail with reference to FIGS. 5 and 6.

FIG. 5 is a schematic cross-sectional view for explaining a flow of air that is introduced into and discharged from the pad-type magnetic stimulation device 1000 of the present invention. FIG. 5 illustrates a cross-section taken in a lateral direction in a state in which the magnetic field generating coil 100 is seated on the guide unit 300, and the case unit 200 is coupled thereto.

As described above, the air blowing device 420 may be disposed below the through-hole 312 of the case unit 300. The air blowing device 420 generates an airflow within the inner space, and the airflow is illustrated by solid-line arrows. According to the illustrated path, the air introduced from the lower side surface of the magnetic field generating coil 100 flows sequentially through the upper surface and the lower surface of the magnetic field generating coil 100 and then is discharged to the lower side surface of the magnetic field generating coil 100.

The external air is introduced to one side of the lower case unit 220 through the above-described first vent 411. The first vertical part 320 of the guide unit 300 guides the air so that the air introduced from the lower case unit 220 flows toward the upper surface of the magnetic field generating coil 100. In other words, the first vertical part 320 serves to prevent the air introduced from the lower case unit 220 from being introduced toward the lower surface of the magnetic field generating coil 100. The air guided toward the upper surface of the magnetic field generating coil 100 by the first vertical part 320 cools the space between the magnetic field generating coil 100 and the body contact surface 211. Thereafter, the second vertical part 330 guides the air so that the air passing through the upper surface of the magnetic field generating coil 100 moves from the central area of the magnetic field generating coil 100 toward the lower surface of the magnetic field generating coil 100. The air introduced toward the lower surface of the magnetic field generating coil 100 sweeps over the lower surface through the protruding ribs 311. Thereafter, the air is discharged to the outside through the through-holes 312 formed in the bottom surface 310.

The second vertical part 330 may be disposed adjacent to a rear surface of the upper case unit 210 so that a negative pressure is generated in the inner space through the air blowing device 420.

FIG. 6 is a schematic cross-sectional view for explaining a flow of air that is introduced into and discharged from the pad-type magnetic stimulation device 1000 of the present invention.

As described above, the pad-type magnetic stimulation device 1000 of the present invention includes a first vent 411 through which external air is introduced into an inner space and a second vent 412 through which the air in the inner space is discharged to the outside. Here, since the first vent 411 and the second vent 412 are formed at positions that are misaligned with each other, the flows of the air at the introduction side and the discharge side are misaligned with each other as illustrated in FIG. 6.

Referring to FIG. 6, the air introduced from the lower side surface of the magnetic field generating coil 100 flows sequentially through the upper surface and the lower surface of the magnetic field generating coil 100 and then is discharged from the lower side surface of the magnetic field generating coil 100. Here, it is preferable that the air introduced into one side of the magnetic field generating coil is discharged to the other side, and the inflow and outflow paths are spaced apart from each other as illustrated.

Hereinafter, the magnetic field generating coil 100 will be described in more detail with reference to FIGS. 7 to 12.

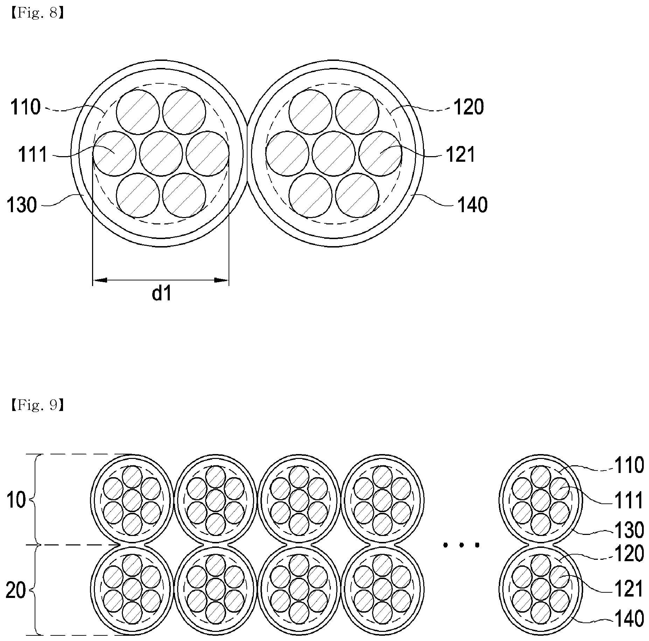

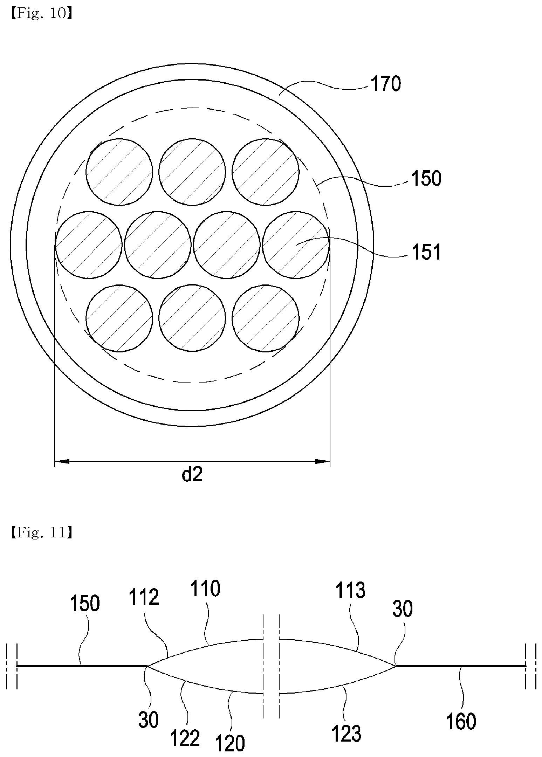

FIG. 7 is a perspective view of the magnetic field generating coil 100 included in the pad-type magnetic stimulation device 1000 of the present invention, FIG. 8 is a cross-sectional view taken along line A-A' of the magnetic field generating coil, FIG. 9 is a cross-sectional view taken along line B-B' of the magnetic field generating coil, and FIG. 10 is a cross-sectional view taken along line C-C' of the magnetic field generating coil, and FIG. 11 is a schematic view for explaining a structure of the magnetic field generating coil.

Referring to FIG. 8, the magnetic field generating coil 100 includes coil bundles 110 and 120 including unit coil strands 111 and 121 made of a copper material, and hollow tubes 130 and 140 surrounding the coil bundles 110 and 120. The magnetic field generating coil 100 may be a disc-shaped coil in which the coil bundles 110 and 120 and the hollow tubes 130 and 140 are wound to be integrated with each other.

The coil bundles 110 and 120 include a first coil bundle 110 and a second coil bundle 120 connected in parallel to the first coil bundle 110.

The coil bundles 110 and 120 is provided by gathering a plurality of unit coil strands 111 and 121. The first coil bundle 110 is provided by gathering a plurality of unit coil strands 111, and the second coil bundle 120 is provided by gathering a plurality of unit coil strands 121.

Here, it is preferable that each of the unit coil strands 111 and 121 is provided as an oxygen free copper (OFC) coil having a low oxygen-to-copper ratio.

The OFC coil means a high-purity copper wire with a purity of 99.9% or higher, containing no oxides other than copper. The higher the oxygen content in the coil, the more likely it is that the current flow is interrupted. Conversely, the higher the copper purity, the smoother the current flow. The OFC coil used in the magnetic field generating coil 100 has a lower oxygen ratio than that of a general coil and thus has lower resistance or impedance, so the magnetic field generating coil 100 has an advantage of low heat generation.

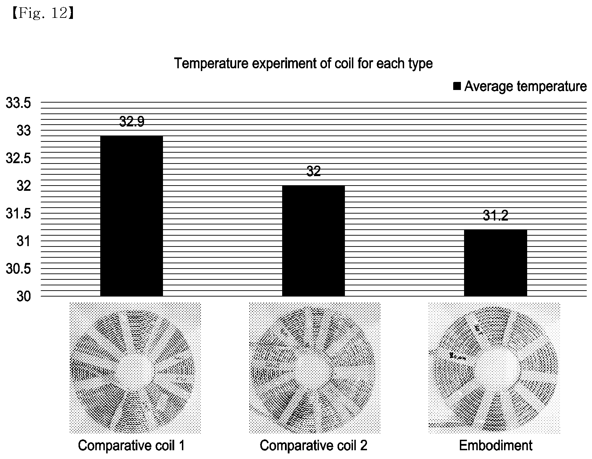

It is preferable that a coil selected between AWG 8 and AWG 12, which is a standard specification of a wire diameter (or cross-sectional area) is used as the OFC coil used in the magnetic field generating coil 100. When the OFC coil having a diameter within the above-described range is selected and used, it may be confirmed that heat generation is controlled without separate cooling while performing the non-invasive treatment using the magnetic field generating coil 100 of the present invention. Specific heat control performance will be described later with reference to FIG. 12.

The hollow tubes 130 and 140 include a first tube 130 that surrounds the first coil bundle 110 and a second tube 140 that surrounds the second coil bundle 120.

The first tube 130 surrounds the first coil bundle 110, and the second tube 140 surrounds the second coil bundle 120. The hollow tubes 130 and 140 may be PVC tubes made of a flexible material. The hollow tubes 130 and 140 serve to encapsulate the coil bundles 110 and 120.

Here, the first tube 130 and the second tube 140 may be in close contact with each other and be at least partially connected to each other. As illustrated in FIG. 2, in the first tube 130 and the second tube 140, outer circumferences of the first tube 130 and the second tube 140 may be partially connected to each other so that a cross-section thereof has a figure-8 shape. The first tube 130 and the second tube 140 may be formed in a form in which the first tube 130 and the second tube 140 are in close contact with each other in a longitudinal direction.

The magnetic field generating coil 100 may be a disc-shaped coil in which the coil bundles 110 and 120 and the hollow tubes 130 and 140 are wound to be integrated with each other, as illustrated in FIG. 7.

Here, a diameter D1 of the magnetic field generating coil 100 may be 100 mm to 300 mm. More preferably, the first diameter D1 may be 150 mm to 250 mm.

The disc-shaped magnetic field generating coil 100 may be formed in a two-layer structure. Referring to FIG. 9, the magnetic field generating coil 100 includes a first layer 10 and a second layer 20. The first coil bundle 110 and the second coil bundle 120 described above may form the first layer 10 and the second layer 20 of the disc-shaped coil, respectively.

The magnetic field generating coil 100 further includes an input-side coil bundle 150 and an output-side coil bundle 160.

The first coil bundle 110 forming the first layer 10 and the second coil bundle 120 forming the second layer 20 of the disc-shaped magnetic field generating coil 100 may be connected in parallel to the input-side coil bundle 150 and the output-side coil bundle 160.

Referring to FIG. 11, the input-side coil bundle 150 is connected to a first end 112 of the first coil bundle 110 and a first end 122 of the second coil bundle 120. The output-side coil bundle 160 is connected to the second end 113 of the first coil bundle 110 and the second end 123 of the second coil bundle 120.

Here, it is preferable that a diameter d2 of the input-side coil bundle 150 is larger than the diameter D1 of the first coil bundle 110.

In addition, the diameter of the output-side coil bundle 160 may be the same as the diameter d2 of the input-side coil bundle 150, and the diameter of the second coil bundle 120 may be the same as the diameter D1 of the first coil bundle 110.

A connection part 30 may be provided at a point connecting the input-side coil bundle 150 to the first coil bundle 110 and the second coil bundle 120 and/or at a point connecting the output-side coil bundle 160 to the first coil bundle 110 and the second coil bundle 120. The connection part 30 electrically connects the input-side coil bundle 150, the first coil bundle 110, and the second coil bundle 120 to each other.

The input-side tube 170 surrounds the input-side coil bundle 150. The input-side tube 170 may be a hollow tube like the first tube 130 and the second tube 140, and may be a flexible PVC tube. The hollow input-side tube 170 serves to encapsulate the input-side coil bundle 150. Likewise, an output-side tube 180 may be provided, and the output-side tube 180 surrounds the output-side coil bundle 160.

The magnetic field generating coil 100 of the may further include a support part 40. Referring to FIG. 7, the support part 40 serves to support the disc-shaped coil in which the coil bundles 110 and 120 and the hollow tubes 130 and 140 are wound to be integrated with each other. The support part 40 may be a belt-shaped member capable of simultaneously surrounding and supporting coils, which are wound in a plurality of layers, of the magnetic field generating coil 100.

The conditions of current, voltage, frequency, etc., applied to the magnetic field generating coil 100 are exemplified as follows.$Current = 500 ∼ 900 A$$Voltage = 100 ∼ 1000 V$$Frequency = 1 ∼ 100 Hz$

However, it should be noted that these figures are exemplary and do not limit the scope of the present invention.

FIG. 12 is a graph obtained by comparing results of a heat generation temperature of a magnetic field generating coil that is another type of magnetic field generating coil according to an embodiment of the present invention.

An illustrated comparative coil 1 is a solid-type copper coil used in the conventional magnetic field generating coil, and a comparative coil 2 is a coil that has a shape in which a coil bundle formed by gathering a plurality of unit coil strands is wound, like the coil of the present invention, but is made of a tin material. The coil of the embodiment corresponds to the magnetic field generating coil 100 of the present invention.

The comparative coil 1, the comparative coil 2, and the coil according to the embodiment have the same conductor diameter of 12AWG, and the wound disc has an inner diameter of Ø90 and an outer diameter of Ø300. At room temperature of 26°C, current was applied to each coil, and temperatures were measured three times after approximately one hour. A shown temperature represents a mean value obtained through the three measurements.

While a temperature of the comparative coil 1 and a temperature of the comparative coil 2 rapidly increased to 32.9°C and 32°C, respectively, the magnetic field generating coil 100 of the present invention showed a lower temperature rise of 31.2°C compared to the comparative coil 1 and the comparative coil 2.

A temperature difference of approximately 1°C as described above may have a significant effect on heat control performance. Since magnetic treatment is performed for more than 20 minutes using the magnetic field generating coil, the magnetic field generating coil 100 of the present invention, which shows the lower temperature rise compared to the comparative coils, may reduce heat generated from the coil during that magnetic treatment lasting several tens of minutes and maximize magnetic field generation efficiency.

In the above, the magnetic field generating coil 100 disclosed in the present invention is exemplified as having the overall circular shape, but is not limited thereto. The magnetic field generating coil 100 may be a coil wound to have various shapes such as an oval, figure 8, disc, or polygon when viewed from the vertical upper or lower portions.

Although the embodiment of the inventive concept is described with reference to the accompanying drawings, those with ordinary skill in the technical field of the inventive concept pertains will be understood that the present disclosure can be carried out in other specific forms without changing the technical idea or essential features. Therefore, the above-disclosed embodiments are to be considered illustrative and not restrictive.

## Claims

1. A pad-type magnetic stimulation device, which applies a magnetic field to nerve cells or muscle cells of a body to provide non-invasive stimulation, comprising:
a pad-type case unit comprising an upper case unit having a body contact surface and a lower case unit coupled to a lower portion of the upper case unit to form an inner space;
a magnetic field generating coil provided in the inner space to generate a magnetic field;
a cooling unit configured to cool the magnetic field generating coil by generating an airflow in the inner space; and
a guide unit configured to provide a space, in which the magnetic field generating coil is seated, and guide the airflow in the vicinity of the magnetic field generating coil.

2. The pad-type magnetic stimulation device of claim 1, wherein the guide unit is configured to guide air introduced from a first side surface of the lower case unit so that the air sequentially flows along an upper surface of the magnetic field generating coil and a lower surface of the magnetic field generating coil and then is discharged through a second side surface of the lower case unit.

3. The pad-type magnetic stimulation device of claim 2, wherein the guide unit comprises:
a bottom surface on which the magnetic field generating coil is seated;
a first vertical part which is formed to have a predetermined height along an outer circumference of the bottom surface and surrounds an outer circumference of the magnetic field generating coil; and
a second vertical part which is formed to have a predetermined height at a central area of the bottom surface and is configured to support an inner circumference of the magnetic field generating coil.

4. The pad-type magnetic stimulation device of claim 3, wherein the guide unit comprises a protruding rib protruding from the bottom surface to support the lower surface of the magnetic field generating coil so that the magnetic field generating coil is spaced apart from the bottom surface.

5. The pad-type magnetic stimulation device of claim 3, wherein the guide unit comprises a through-hole formed to pass through the bottom surface in at least partial area of the bottom surface.

6. The pad-type magnetic stimulation device of claim 5, wherein the cooling unit comprises:
a first vent formed at one side of the lower case unit to introduce external air into the inner space;
a second vent formed at the other side of the lower case unit to discharge the air in the inner space to the outside; and
an air blowing device disposed at a side of the second vent to discharge the air in the inner space,
wherein the air blowing device is disposed below the through-hole.

7. The pad-type magnetic stimulation device of claim 3, wherein, when the upper case unit and the lower case unit are coupled to each other, the second vertical part is in contact with a rear surface of the upper case unit.
